# EUROPÄISCHE PATENTSCHRIFT

(11) **EP 0 758 214 B1**
(45) Veröffentlichungstag und Bekanntmachung des Hinweises auf die Patenterteilung: **20.02.2002**
(21) Anmeldenummer: 95917265.1
(22) Anmeldetag: 04.05.1995
(51) Int. Cl.: A61B 17/128, A61B 17/122, A61B 17/02

(54) **INSTRUMENT ZUM EINSATZ BEI ENDOSKOPISCHEN EINGRIFFEN**
INSTRUMENT FOR USE IN ENDOSCOPIC OPERATIONS
INSTRUMENT UTILISE POUR DES INTERVENTIONS ENDOSCOPIQUES

(30) Priorität: 04.05.1994 DE 4415521
(43) Veröffentlichungstag der Anmeldung: 19.02.1997
(60) Teilanmeldung: 01116327.6
(73) Patentinhaber: Erbengemeinschaft Dr. h.c. Karl Storz, vertreten durch Frau Storz-Reling, Sybill, 78532 Tuttlingen (DE)
(72) Erfinder: STORZ, Karl, (DE); CUSCHIERI, Alfred, Dundee DD1 9SY (GB)
(74) Vertreter: Münich, Wilhelm, Dr.
(86) Internationale Anmeldenummer: DE9500577
(87) Internationale Veröffentlichungsnummer: WO9530376

(56) Entgegenhaltungen:
- WO-A-92/13490
- DE-A- 3 044 186
- US-A- 4 337 774
- US-A- 4 706 668
- US-A- 4 957 500
- US-A- 5 304 183

## Beschreibung

### Technisches Gebiet

Die Erfindung bezieht sich auf ein Instrument zum Einsatz bei endoskopischen Eingriffen am menschlichen oder tierischen Körper.

Endoskopische Eingriffe werden seit einigen Jahren immer häufiger anstelle von "offenen chirurgischen Eingriffen" ausgeführt. Dabei werden durch einen Kanal eines Einsetzinstruments, beispielsweise eines Arbeitstrokars, die eigentlichen Arbeitsinstrumente, wie Endoskope, Zangen, Scheren usw. in den Körper eingesetzt.

### Stand der Technik

Bei einer Reihe von endoskopischen Eingriffen werden sog. Rohrschaftinstrumente, wie Zangen, Scheren, Clipapplikatoren etc. verwendet, die in den Kanal eines Trokars für die DurcHführung des jeweiligen Eingriffs eingesetzt werden. Für eine Reihe von Operationen werden Trokare mit einem Kanal verwendet, dessen lichte Weite nur ca. 5 mm beträgt. Damit ist es in der Regel nicht möglich, in den Kanal eines Trokars gleichzeitig zwei Instrumente einzusetzen. Dies bedeutet, daß bei der Verwendung eines herkömmlichen endoskopischen Instrumentariums immer dann ein weiterer Zugang geschaffen werden muß, wenn es beispielsweise während des Operationsvorgangs erforderlich ist, periphere Arterien mittels einer Klemme zu verschließen.

Gemäß der US-A-5 304 183 wird als nächstliegender Stand der Technik ein Instrument zum Einsatz bei endoskopischen Eingriffen am menschlichen oder tierischen Körper offenbart, das ein Einsetzinstrument, das in den menschlichen oder tierischen Körper einsetzbar ist, und das einen Kanal aufweist, und ein Arbeitsinstrument aufweist, das durch den Kanal des Einsetzinstruments in den Körper einbringbar ist, und das wenigstens aus einem distal angeordneten Arbeitselement und einem länglichen Einführungs- und Betätigungsteil besteht, der den Kanal durchsetzt und der das Arbeitselement mit dem proximalen Bereich des Instruments verbindet, so dass das Arbeitselement vom proximalen Bereich aus betätigt werden kann, wobei das Arbeitselement und das Einführungs- und Betätigungsteil über einen Verbindungsmechanismus derart verbunden sind, dass das Arbeitselement vom Einführungs-und Betätigungsteil intrakorporal lösbar ist, so dass der Betätigungsteil ohne Arbeitselement aus dem Kanal entnommen werden kann, das Arbeitselement nach der Trennung vom Einführungs-und Betätigungsteil wenigstens in einem Funktionszustand verbleibt, und das Arbeitselement nach dem Wiedereinsetzen des Einführungs- und Betätigungsteils in den Kanal des Einsetzinstruments wieder mit diesem verbunden und anschließend durch den Kanal entnommen werden kann.

Der aus der US-A-5 304 183 bekannte Verbindungsmechanismus zwischen distalem Arbeitselement und dem länglichen Einführungs- und Betätigungsteil ist als eine Zapfen-Loch-Verbindung ausgebildet, die eine sehr genaue Querbewegung bei der Wiederverriegelung der Elemente erfordert. Damit ist die Herstellung der Verbindung erschwert. Zudem besteht die Möglichkeit, dass das Arbeitselement unbeabsichtigt im Körper verloren wird.

Gemäß der DE-A-30 44 186 wird ein Klammerhaltegerät zum Abklemmen von Blutgefäßen offenbart, wobei ein Arbeits-element als eine Klammer mit Alpha-Form ausgebildet ist, die in einem "V" endet. Dabei besteht die Klammer aus hinteren Schenkeln, die von einer abgerundeten Spitze unter elastischer Spannung auseinanderlaufen, sich dann kreuzen und in im wesentlichen geraden Schenkeln enden, die den Greifabschnitt der Klammer bilden.

Der Vorgang des Zusammenspiels zwischen der Klammer und dem Haken erfolgt folgendermaßen:

Die Klammer wird über ihre abgerundete Spitze am Ende (Haken) angehakt. D.h. erstens der Haken greift in die Klammer selbst ein - es liegt also keine explizite Öse vor - und zweitens gibt es nur zwei Stellungen (eine Ausgangsstellung und eine zweite bei Drehung des Hakens um 180° um die Längsachse des Stabes an dem sich der Haken befindet) die Haken und Klammer überhaupt zueinander einnehmen können. Die Verbindung beider Elemente wird damit nur in diesen beiden möglichen Stellungen hergestellt oder gelöst. Wird der Haken um die Längsachse gedreht, so dreht sich die angehakte Klammer mit.
Ein Sichern der Verbindung durch ein gegenseitiges Verdrehen von Haken und Klammer wie bei einer Schlüsselloch-Verbindung ist nicht möglich. Die Klammer wird nur durch ein Rohr sicher am Haken gehalten. Befindet sich der Haken außerhalb des Rohrs 13, so ist die Verbindung Haken zu Klammer nicht gesichert. Diese Art der Verbindung kann dazu führen, dass das Arbeitselement unbeabsichtigt im Körper verloren wird.

### Darstellung der Erfindung

Der Erfindung liegt die Aufgabe zugrunde, ein Instrument zum Einsatz bei endoskopischen Eingriffen am menschlichen oder tierischen Körper zu schaffen, das während eines Eingriffs stationär eine bestimmte Aufgabe ausführt, ohne daß für den Einsatz dieses Instruments ein weiterer Zugang zu dem Hohlraum im menschlichen oder tierischen Körper, in dem der Eingriff ausgeführt wird, vorhanden sein müßte. Zudem soll der Verbindungsmechanismus zwischen distalem Arbeits-Element und länglichem Einführungs- und Betätigungsteil wie gemäß dem Stand der Technik derart weitergebildet werden, dass ein solches Instrument gemäß der US-A-5 304 183 im intrakorporalen Raum einfach und sicher zu betätigen ist.

Eine erfindungsgemäße Lösung dieser Aufgabe ist im Anspruch 1 angegeben. Erfindungsgemäß besteht das Instrument wenigstens aus einem distal angeordneten Arbeitselement und einem länglichen Einführungs- und Betätigungsteil, der im das Arbeitselement mit dem proximalen Bereich verbindet. Das Arbeitselement und das Einführungs- und Betätigungsteil sind über einen Verbindungsmechanismus derart verbunden, daß das Arbeitselement intrakorporal vom Einführungs- und Betätigungsteil lösbar und wieder mit diesem verbindbar ist.

Die Aufgabe wird insbesondere dadurch gelöst, dass ein Verbindungsmechanismus vorgesehen ist, wobei die Verbindung durch eine einfache axiale und eine danach folgende unkritische Schließ-Bewegung sichergestellt wird. Gemäß dem Hauptanspruch 1 ist der Verbindungsmechnismus als eine verzahnte Zange ausgebildet.

Hierdurch ist es möglich, zunächst das Arbeitselement des erfindungsgemäßen Instruments beispielsweise durch den Kanal eines als Einsetzinstruments dienenden Trokars einzusetzen, das Arbeitselement im Körperinneren zu positionieren und anschließend das Einführungs- und Bedienteil vom Arbeitselement zu lösen. Das Einführungs- und Bedienteil kann dann aus dem Kanal des Trokars entnommen werden, so daß nunmehr ein weiteres Arbeitsinstrument, wie beispielsweise eine Zange oder eine Schere oder ein Endoskop eingesetzt werden kann. Nach Abschluß der Arbeiten mit dem weiteren Arbeitsinstrument und nach der Funktionserfüllung des in das Körperinnere eingesetzten Arbeitselements wird dieses wieder mit dem Einführungs- und Bedienteil verbunden und aus dem Körperinneren entnommen. Selbstverständlich ist es aber auch möglich, das Arbeitselement nach der erneuten Verbindung mit dem Einführungs- und Bedienteil im Körperinneren neu zu positionieren und somit an einer anderen Stelle weiterzuverwenden.

Dabei soll das Arbeitselement nach der Trennung vom Einführungs- und Betätigungsteil wenigstens in einem Funktionszustand verbleiben. Wenn beispielsweise der distale Teil des Instruments eine Klemme ist, die im nicht betätigten Zustand geschlossen ist, kann die Klemme vor Beginn des eigentlichen Operationsvorgangs eingesetzt und so positioniert werden, daß sie z.B. eine periphere Arterie "abklemmt". In diesem Falle ist es bevorzugt, wenn die Klemme in Art einer Buldoc-Klemme ausgebildet ist, wie sie bei offenen Operationen eingesetzt wird. Nach Beendigung des eigentlichen Operationsvorgangs wird dann die Klemme wieder entnommen.

Der Verbindungsmechanismus, der das Arbeitselement und das Einführungs- und Betätigungsteil miteinander lösbar verbindet, ist folgendermaßen ausgebildet:

Der Verbindungsmechanismus weist eine am Einführungs- und Betätigungsteil angebrachte Zange auf, deren Zangenmaul durch Betätigung des Bedienteils geöffnet und geschlossen werden kann, wobei deren Zangenelemente die gleiche Zahnung wie die gegriffenen Flächen des Arbeitselements haben kann. Hierdurch ist sichergestellt daß das Arbeitselement von der Zange "rutsch- und verdrehsicher" gegriffen wird. Die Zahnung kann dabei insbesondere als Karo ausgebildet ist, dessen einzelne Zähne beispielsweise pyramidenförmig gestaltet sind.

Der distale Teil des erfindungsgemäßen Instruments, d.h. das Arbeitselement kann selbstverständlich nicht nur als Klemme ausgebildet sein. Als Arbeitselement können vielmehr die verschiedensten Instrumente verwendet werden, wie sie für die Durchführung stationärer Aufgaben während eines Eingriffs bekannt sind. Beispielsweise kann der distale Teil ein Rückhalter oder ein Retractor, z.B. ein Leberretractor sein.

Ein Leberretractor besteht aus einem "Drahtkäfig", der sich nach dem Einsetzen des Instruments in den Hohlraum "öffnet". Durch Einziehen in den Einführungsteil wird der Retractor geschlossen.

Selbstverständlich ist es auch möglich, Arbeitselemente zu verwenden, die nach der Trennung vom Einführungs- und Betätigungsteil zumindest zwei stabile Funktionszustände einnehmen können. Derartige Arbeitselemente können beispielweise die Funktionszustände "aktiv" und "passiv" haben. Um das Arbeitselement ohne aktive Stellelemente, wie beispielsweise elektromechanische Elemente vom passiven in den aktiven Funktionszustand zu überführen, weist bei einer bevorzugten Weiterbildung das Arbeitselement mindestens ein Stellelement auf, das aus einem Werkstoff mit Formgedächtnis besteht. Ferner können auch superelastische Materialien etc. zum Einsatz kommen.

Weiterhin ist es bevorzugt, wenn der Verbindungsmechanismus zumindest axiale Zug- und Druckkräfte auf das Arbeits-element übertragen kann, da hierdurch die meisten Funktionen üblicher Arbeitselemente gesteuert werden können, und insbesondere eine einfache Überführung der Elemente in den jeweils gewünschten Funktionszustand möglich ist.

Durch die Weiterbildung, gemäß der der Verbindungsmechanismus einen Schutz gegen unbeabsichtigtes Lösen der Verbindung aufweist, ist sichergestellt, daß das Arbeitselement nicht unbeabsichtigt im Körper verloren wird.

Bevorzugt ist es ferner, wenn der Einführungs- und Betätigungsteil aus einem stabförmigen Teil, der in den Kanal des Einsetzinstruments einsetzbar ist, und einem proximalen Bedienteil besteht. Hierdurch hat das erfindungsgemäße Instrument den gleichen Grundaufbau wie herkömmliche Instrumente, so daß die Bedienungsperson bei der Bedienung nicht grundsätzlich "umlernen" muß. Der proximale Bedienteil kann in an sich bekannter Weise von dem stabförmigen Teil gelöst werden, wie dies von Instrumenten mit der Bezeichnung "Take apart" der Karl Storz GmbH & Co. bekannt ist. Hierdurch wird nicht nur die Reinigung erleichtert, sondern es ist auch möglich, andere Bedienteile, wie beispielsweise anders geformte Handgriffe anzusetzen, wenn einem Benutzer ein Handgriff nicht "zusagt".

Ferner ist es möglich, das Arbeitselement durch Rückzug in den Einführungsteil zu versteifen. Hierdurch wird der Einsetz- und der Entnahmevorgang vereinfacht und darüberhinaus die Gefahr verringert, daß das Arbeitselement versehentlich verloren wird.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

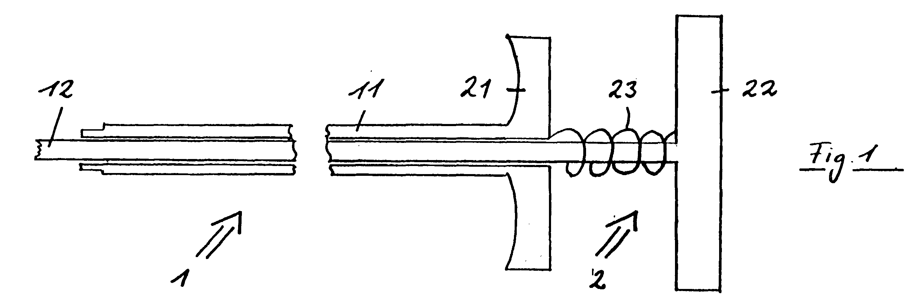

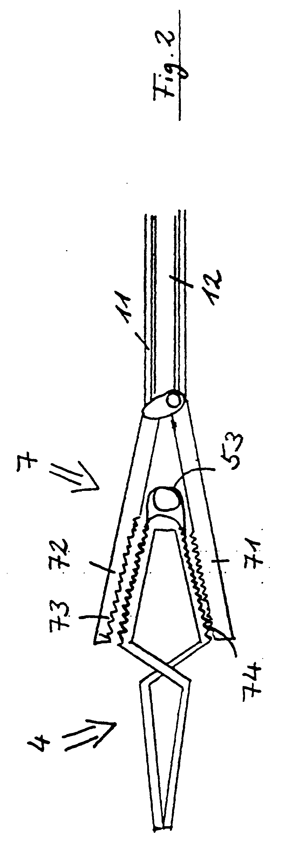

- Fig. 1+2: den Einführungsteil und den distalen Teil eines Ausführungsbeispiels,

### Beschreibung von Ausführungsbeispielen

Bei dem in Figur 1 dargestellten Ausführungsbeispiel ist das Arbeitselement des Instruments eine Klemme, die im nicht betätigten Zustand geschlossen ist, und die insbesondere in Art einer Buldoc-Klemme ausgebildet ist.

Der in Fig. 1 dargestellte Einführungs- und Betätigungsteil besteht aus einem stabförmigen Teil 1, der in den Kanal eines nicht dargestellten Einsetzinstruments, wie eines Trokars einsetzbar ist, und einem proximalen Bedienteil 2. Der stabförmige Teil 1 wiederum besteht aus einem zylindrischen Rohr 11 und einem Stab 12, der in dem Rohr 11 in Richtung seiner Längsachse verschiebbar ist. Der Bedienteil 2 besteht aus zwei Handgriffen 21 und 22, von denen der Handgriff 21 mit dem Rohr 11 und der Handgriff 22 mit dem Stab 12 verbunden ist. Zwischen die Handgriffe 21 und 22 ist eine Feder 23 eingesetzt.

In Fig. 2 sind jeweils gleiche oder entsprechende Teile mit den selben Bezugszeichen bezeichnet, so daß auf eine erneute Vorstellung verzichtet wird, und lediglich die Abweichungen des in dieser Figur dargestellten Ausführungsbeispiels.

Gemäß Fig. 2 weist der Verbindungsmechanismus eine am stabförmigen Teil 1 angebrachte Zange 7 auf, deren durch Zangenelemente 71 und 72 gebildetes Zangenmaul durch Betätigung des Bedienteils 2 geöffnet und geschlossen werden können. Die Zangenelemente 71 und 72 haben an den Greifflächen die gleiche Zahnung 73 wie die gegriffenen Flächen 74 der Buldoc-Klemme 4, die bei der gezeigten Modifikation einstückig ausgeführt ist. Die Zahnung 73 ist als Karo ausgebildet ist, dessen einzelne Zähne pyramidenförmig gestaltet sind. Anstelle der dargestellten Feder 53 kann die Buldoc-Klemme auch eine Schenkelfeder haben.

Vorstehend ist die Erfindung ohne Beschränkung des allgemeinen Erfindungsgedankens beschrieben worden, wie er den Ansprüchen entnehmbar ist.

## Patentansprüche

1. Instrument zum Einsatz bei endoskopischen Eingriffen am menschlichen oder tierischen Körper, das
- ein Einsetzinstrument (11), das in den menschlichen oder tierischen Körper einsetzbar ist, und das einen Kanal hat, und
- ein Arbeitsinstrument (12) aufweist, das durch den Kanal des Einsetzinstruments in den Körper einbringbar ist, und das
- wenigstens aus einem distal angeordneten Arbeitselement (4), und
- einem länglichen Einführungs- und Betätigungsteil (1, 2) besteht, der den Kanal durchsetzt und der das Arbeitselement (4) mit dem proximalen Bereich des Instruments verbindet, so dass das Arbeitselement (4) vom proximalen Bereich aus betätigt werden kann,
wobei das Arbeitselement (4) und das Einführungs- und Betätigungsteil (1, 2) über einen Verbindungsmechanismus derart verbunden sind,
dass das Arbeitselement (4) vom Einführungs- und Betätigungsteil (1, 2) intrakorporal lösbar ist, so dass der Betätigungsteil (2) ohne Arbeitselement (4) aus dem Kanal entnommen werden kann,
das Arbeitselement (4) nach der Trennung vom Einführungs-und Betätigungsteil (1, 2) wenigstens in einem Funktionszustand verbleibt, und
das Arbeitselement (4) nach dem Wiedereinsetzen des Einführungs- und Betätigungsteils (1, 2) in den Kanal des Einsetzinstruments wieder mit diesem verbunden und anschließend durch den Kanal entnommen werden kann, wobei der Verbindungsmechanismus eine am Einführungs- und Betätigungsteil (1, 2) angebrachte Zange (7) aufweist, deren Zangenmaul durch Betätigung des Betätigungsteils (2) geöffnet und geschlossen werden kann, **dadurch gekennzeichnet, dass** die Zangenelemente (71, 72) und die gegriffenen Flächen (74) des Arbeitselements (4) eine gleiche Zahnung (73) aufweisen, die sicherstellt, dass das Arbeitselement (4) von der Zange (7) rutsch- und verdrehsicher gegriffen wird.

2. Instrument nach Anspruch 1,
**dadurch gekennzeichnet, daß** das Arbeitselement (4) nach der Trennung vom Einführungs- und Betätigungsteil (1,2) zumindest zwei stabile Funktionszustände einnehmen kann.

3. Instrument nach einem der Ansprüche 1 oder 2,
**dadurch gekennzeichnet, daß** der Verbindungsmechanismus zumindest axiale Zug- und Druckkräfte auf das Arbeitselemen (4) übertragen kann.

4. Instrument nach einem der Ansprüche 1 bis 3,
**dadurch gekennzeichnet, daß** der Verbindungsmechanismus einen Schutz gegen unbeabsichtigtes Lösen der Verbindung aufweist.

5. Instrument nach einem der Ansprüche 1 bis 4,
**dadurch gekennzeichnet, daß** der Einführungs- und Betätigungsteil (1,2) aus einem stabförmigen Teil (12), der in den Kanal des Einsetzinstruments (11) einsetzbar ist, und einem proximalen Bedienteil (2) besteht.

6. Instrument nach Anspruch 5,
**dadurch gekennzeichnet, daß** der proximale Bedienteil (2) von dem stabförmigen Teil (12) gelöst werden kann.

7. Instrument nach Anspruch 5 oder 6,
**dadurch gekennzeichnet, daß** das Arbeitselement (4) durch Rückzug in den Einführungsteil (1) versteift wird.

8. Instrument nach einem der Ansprüche 1 bis 7,
**dadurch gekennzeichnet, daß** das Arbeitselement (4) mindestens ein Stellelement (53) aufweist, das aus einem Werkstoff mit Formgedächtnis besteht.

9. Instrument nach einem der Ansprüche 1 bis 8,
**dadurch gekennzeichnet, daß** das Arbeitselement (4) eine Klemme ist, die im nicht betätigten Zustand geschlossen ist.

10. Instrument nach Anspruch 9,
**dadurch gekennzeichnet, daß** die Klemme in Art einer Buldoc-Klemme und insbesondere als einstückige Buldoc-Klemme ausgebildet ist.

11. Instrument nach einem der Ansprüche 1 bis 8,
**dadurch gekennzeichnet, daß** das Arbeitslement (4) ein Retractor ist.

12. Instrument nach Anspruch 11,
**dadurch gekennzeichnet, daß** der Retractor durch Einziehen in den Einführungsteil geschlossen wird.

13. Instrument nach Anspruch 11 oder 12,
**dadurch gekennzeichnet, daß** der Retractor ein Leberretractor ist.

14. Instrument nach einem der Ansprüche 1 bis 13,
**dadurch gekennzeichnet, daß** die Zahnung (73) als Karo ausgebildet ist, dessen einzelne Zähne insbesondere pyramidenförmig gestaltet sind.

## Claims

1. Instrument for use in endoscopic operations on the human or animal body, comprising
- an insertion instrument (11) adapted for being inserted into the human or animal body and presenting a duct, and
- an operating instrument (12) adapted to be introduced through said duct of said insertion instrument into the body and
- consisting at least of a distally disposed operating element (4) and
- an elongate insertion and actuating component (1, 2) passing through said duct and connecting said operating element (4) to the proximal region of the instrument such that said operating element (4) may be operated from the proximal region,
with said operating element (4) and said insertion and actuating component (1, 2) being connected via a connecting mechanism in such a way
that said operating element (4) may be detached from said insertion and actuating component (1, 2) inside the body such that said actuating component (2) may be removed from said duct without said operating element,
that said operating element (4) remains at least in one functional state after detachment from said insertion and actuating component (1, 2), and
after re-insertion of said insertion and actuating component (1, 2) into the duct of said insertion instrument, said operating element (4) is adapted for being reconnected to this instrument and subsequently removed through said duct,
wherein said connecting mechanism presents forceps (7) mounted on said insertion and actuating component (1, 2), whose forceps jaws may be opened and closed by actuation of said actuating component (2),
**characterised in that** the forceps elements (71, 72) and the engaged surfaces (74) of said operating element (4) present the same toothing (73) that ensures that said operating element (4) will be engaged by said forceps (7) in a way protected from sliding and twisting.

2. Instrument according to Claim 1,
**characterised in that**, after detachment from said insertion and actuating component (1, 2), said operating element (4) may assume at least two stable functional states.

3. Instrument according to any of the Claims 1 or 2,
**characterised in that** said connecting mechanism is capable of transmitting at least axial tensile or pressure forces to said operating element (4).

4. Instrument according to any of the Claims 1 to 3,
**characterised in that** said connecting mechanism presents provisions counteracting an unintentional interruption of the connection.

5. Instrument according to any of the Claims 1 to 4,
**characterised in that** said insertion and actuating component (1, 2) consists of a rod-shaped part (12), that is adapted for being introduced into the duct of said insertion instrument (11), and of a proximal actuating component (2).

6. Instrument according to Claim 5,
**characterised in that** said proximal actuating component (2) is adapted for detachment from said rod-shaped part (12).

7. Instrument according to Claim 5 or 6,
**characterised in that** said operating element (4) is rigidified by retraction into said insertion component (1).

8. Instrument according to any of the Claims 1 to 7,
**characterised in that** said operating element (4) comprises at least one actuator element (53) consisting of a shape memory material.

9. Instrument according to any of the Claims 1 to 8,
**characterised in that** said operating element (4) is a clamp that is closed in the non-operated condition.

10. Instrument according to Claim 9,
**characterised in that** said clamp is designed in the manner of a Buldoc clamp and particularly in the form of an integral Buldoc clamp.

11. Instrument according to any of the Claims 1 to 8,
**characterised in that** said operating element (4) is a retractor.

12. Instrument according to Claim 11,
**characterised in that** said retractor is closed by pulling it into said insertion component.

13. Instrument according to Claim 11 or 12,
**characterised in that** said retractor is a liver retractor.

14. Instrument according to any of the Claims 1 to 13,
**characterised in that** said toothing (73) is designed in the form of a square or lozenge whose individual teeth present a pyramid shape in particular.

## Revendications

1. Instrument utilisé dans des interventions endoscopiques dans le corps humain ou animal, comprenant
- un instrument d'introduction (11) apte à être introduit dans le corps humain ou animal et présentant un passage, et
- un instrument d'opération (12) apte à être introduit dans le passage dudit instrument d'introduction dans le corps, et
- consistant en au moins un élément d'opération (4) disposé du côté distal, et
- un composant d'introduction et d'actionnement allongé (1, 2) passant à travers ledit passage et raccordant ledit élément d'opération (4) à la région proximale de l'instrument d'une façon, que ledit élément d'opération (4) peut être actionné à partir de la région proximale,
ledit élément d'opération (4) et ledit composant d'introduction et d'actionnement (1, 2) étant raccordé via un mécanisme de connexion d'une manière,
que ledit élément d'opération (4) peut être détaché dudit composant d'introduction et d'actionnement (1, 2) à l'intérieur du corps, et ainsi ledit composant d'actionnement (2) peut être enlevé dudit passage sans ledit élément d'opération,
que ledit élément d'opération (4) reste en au moins un état fonctionnel après son détachement dudit composant d'introduction et d'actionnement (1, 2), et
après l'introduction répétée dudit composant d'introduction et d'actionnement (1, 2) dans le passage dudit instrument d'introduction, ledit élément d'opération (4) est apte à être re-raccordé à cet instrument et ensuite enlevé à travers ledit passage,
dans lequel ledit mécanisme de connexion présente des forceps (7) montés sur ledit composant d'introduction et d'actionnement (1, 2), dont la bouche de forceps peut être ouverte et fermée en actionnant sur ledit composant d'actionnement (2),
**caractérisé en ce que** lesdits éléments de forceps (71, 72) et les surfaces prises (74) dudit élément d'opération (4) présente la même denture (73) qui assure, que ledit élément d'opération (4) sera pris par lesdits forceps (7) d'une manière protégée contre le glissement et une rotation.

2. Instrument selon la revendication 1,
**caractérisé en ce que**, suivant son détachement dudit composant d'introduction et d'actionnement (1, 2), ledit élément d'opération (4) peut prendre au moins deux états fonctionnels stables.

3. Instrument selon une quelconque des revendications 1 ou 2,
**caractérisé en ce que** ledit mécanisme de connexion est apte à transmettre audit élément d'opération (4) au moins des forces axiales de traction ou de pression.

4. Instrument selon une quelconque des revendications 1 à 3,
**caractérisé en ce que** ledit mécanisme de connexion présente des mesures empêchant une déconnexion involontaire du raccord.

5. Instrument selon une quelconque des revendications 1 à 4,
**caractérisé en ce que** ledit composant d'introduction et d'actionnement (1, 2) consiste en une partie sous forme de barre (12), qui est apte à être introduit dans le passage dudit instrument d'introduction (11), et en un composant d'actionnement proximal (2).

6. Instrument selon la revendication 5,
**caractérisé en ce que** ledit composant d'actionnement proximal (2) est apte à être détaché de ladite partie sous forme de barre (12).

7. Instrument selon la revendication 5 ou 6,
**caractérisé en ce que** la rigidité dudit élément d'opération (4) est augmentée par son retrait dans ledit composant d'introduction (1).

8. Instrument selon une quelconque des revendications 1 à 7,
**caractérisé en ce que** ledit élément d'opération (4) comprend au moins un organe (53) fait d'un matériau à mémoire de forme.

9. Instrument selon une quelconque des revendications 1 à 8,
**caractérisé en ce que** ledit élément d'opération (4) est une pince qui est fermée en état non opératoire

10. Instrument selon la revendication 9,
**caractérisé en ce que** ladite pince est conçue de façon d'une pince du type Buldoc et en particulier sous forme d'une pince intégrale du type Buldoc.

11. Instrument selon une quelconque des revendications 1 à 8,
**caractérisé en ce que** ledit élément d'opération (4) est un rétracteur.

12. Instrument selon la revendication 11,
**caractérisé en ce que** ledit rétracteur est fermé en le tirant dans ledit composant d'introduction.

13. Instrument selon la revendication 11 ou 12,
**caractérisé en ce que** ledit rétracteur est un rétracteur de foie.

14. Instrument selon une quelconque des revendications 1 à 13,
**caractérisé en ce que** ladite denture (73) est conçue sous forme d'un carreau, dont les dents individuelles ont une forme de pyramide en particulier.
